# EUROPEAN PATENT SPECIFICATION

(11) **EP 4 616 800 B1**
(45) Date of publication and mention of the grant of the patent: **04.11.2026**
(21) Application number: 25163910.0
(22) Date of filing: 14.03.2025
(51) Int. Cl.: A61B 5/1486, A61B 5/1495, A61B 5/00

(54) **SYSTEMS AND METHODS FOR SMART BIAS VOLTAGE FOR INSULIN INTERFERENCE COMPENSATION**
SYSTEME UND VERFAHREN FÜR INTELLIGENTE VORSPANNUNGSREGELUNG ZUR KOMPENSATION VON INSULININTERFERENZEN
SYSTÈMES ET PROCÉDÉS POUR UNE TENSION DE POLARISATION INTELLIGENTE POUR UNE COMPENSATION D'INTERFÉRENCE D'INSULINE

(30) Priority: 15.03.2024 US 202463565754 P; 12.03.2025 US 202519077218
(43) Date of publication of application: 17.09.2025
(73) Proprietor: Medtronic MiniMed, Inc., Northridge, CA 91325 (US)
(72) Inventor: Buganski, Meredith H., Northridge, 91325 (US); Rais, Nor Akmaliza, Northridge, 91325 (US); Mahadevan, Aishwarya, Northridge, 91325 (US); Albarracin, Diana C., Northridge, 91325 (US); Namani, Ravi, Northridge, 91325 (US); Chiu, Chia-Hung, Northridge, 91325 (US)
(74) Representative: Zimmermann & Partner Patentanwälte mbB

(56) References cited:
- US-A1- 2020 214 609
- US-A1- 2022 047 187
- US-A1- 2022 125 348

## Description

### CROSS-REFERENCE TO RELATED APPLICATIONS

This application claims the benefit of the filing date of provisional U.S. Patent Application No. 63/565,754 filed on March 15, 2024.

### FIELD

The present disclosure relates generally to continuous glucose monitoring (CGM) and more particularly to analytic sensors and methods for improving interferent rejection and longevity.

### BACKGROUND

Analyte sensors such as biosensors include devices that use biological elements to convert a chemical analyte in a matrix into a detectable signal. There are many types of biosensors used for a wide variety of analytes, including amperometric glucose sensors for glucose level control for diabetes.

A typical glucose sensor works according to the following chemical reactions: 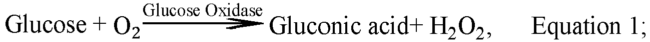 and 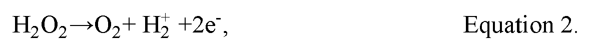 The glucose oxidase is used to catalyze the reaction between glucose and oxygen to yield gluconic acid and hydrogen peroxide, H₂O₂, (Equation 1). The hydrogen peroxide reacts electrochemically as shown in Equation 2, and the current can be measured by a potentiostat. These reactions, which occur in a variety of oxidoreductases known in the art, are used in a number of sensor designs.

One common problem with analyte sensors is that they can electrochemically react not only with the analyte to be measured (or by-product of the enzymatic reaction with the analyte), but can also react with other electroactive chemical species that are not intentionally being measured, which causes an increase in signal strength due to these "interfering species" or "interefrent." Typically, such interfering species are compounds with an oxidation or reduction potential that overlaps with the analyte to be measured (or a by-product of the enzymatic reaction with the analyte). For example, in a conventional amperometric glucose oxidase-based glucose sensor where the conventional sensor measures hydrogen peroxide, interfering species such as acetaminophen, ascorbate, and urate are known to confound true analyte signals, resulting in loss of sensor sensitivity or longevity.

Another common problem is that the analyte sensors are less sensitive when insulin is injected near the sensor. There is room for improvement in the design of analyte sensors to improve sensitivity in the proximity of an insulin bolus.
US 2022/0047187 A1 relates to an application of electrochemical impedance spectroscopy in sensor systems, devices, and related methods. US 2022/0125348 A1 relates to a detection of interferent in analyte sensing.

### SUMMARY

The present invention is directed to an analyte sensor as defined in claim 1, a processor-implemented method as defined in claim 9 as well as one or more non-transitory processor-readable media as defined in claim 14. Further aspects of the invention are defined in dependent claims. The present disclosure relates to analytic sensors and methods for improving interferent rejection and longevity.

In accordance with aspects of the present disclosure, an analyte sensor configured to compensate for insulin interference includes a working electrode including an analyte sensing molecule disposed on the working electrode configured to generate a signal when exposed to an analyte, a processor, and a memory. The working electrode is biased at a first bias voltage value. The memory, includes instructions, which, when executed by the processor, cause the sensor to: obtain an indication from a pump that a bolus is delivered at a point in time; in response to delivery of the bolus, determine at least one of a first electrochemical impedance spectroscopy (EIS) parameter value or a first conductivity value in response to exposure of the working electrode to the analyte; and determine whether an interferent is present based on at least one of the first EIS parameter value or the first conductivity value.

In an aspect of the present disclosure, the instructions, when executed by the processor, may further cause the analyte sensor to adjust the bias voltage of the working electrode from the first bias voltage value to a second bias voltage value in response to determining the presence of the interferent.

In another aspect of the present disclosure, the second bias voltage value may be lower than the first bias voltage value.

In yet another aspect of the present disclosure, the instructions, when executed by the processor, may further cause the analyte sensor to: determine at least one of a second EIS parameter value or a second conductivity value in response to exposure of the electrode to the analyte; and determine the interferent present at an amount is below a threshold amount based on at least one of the second EIS parameter value or the second conductivity value.

In a further aspect of the present disclosure, the instructions, when executed by the processor, may further cause the analyte sensor to adjust the bias voltage of the working electrode in response to determining that the interferent is present in an amount below the threshold amount.

In yet a further aspect of the present disclosure, the adjusting of the bias voltage of the working electrode in response to the interferent being below the threshold amount may include ramping down the bias voltage.

In an aspect of the present disclosure, the first bias voltage may be within a range of insulin excipient oxidation.

In another aspect of the present disclosure, determining that the analyte value is over the threshold may be based on obtaining a size value of the bolus from the pump.

In yet another aspect of the present disclosure, the instructions, when executed by the processor, may further cause the analyte sensor to: prior to the delivery of the bolus obtain the signal from the working electrode indicating an analyte value, determine that the analyte value is over a predetermined threshold; and cause the pump to deliver the bolus in response to the analyte value being over the predetermined threshold.

In a further aspect of the present disclosure, the instructions, when executed by the processor, may further cause the analyte sensor to: prior to the delivery of the bolus obtain an input from a user to cause the pump to deliver the bolus; and cause the pump to deliver the bolus in response the input. The input may include a bolus size.

In accordance with aspects of the disclosure, a processor-implemented method of compensating for insulin interference of an analyte sensor is shown. The method includes: obtaining an indication from a pump that a bolus is delivered at a point in time; in response to the delivery of the bolus, determining at least one of a first electrochemical impedance spectroscopy (EIS) parameter value or a first conductivity value in response to exposure of a working electrode of an analyte sensor to an analyte; and determining a presence of an interferent based on at least one of the first EIS parameter value or the first conductivity value.

In a further aspect of the present disclosure, the method may further include adjusting a bias voltage of the working electrode from a first bias voltage value to a second bias voltage value in response to the presence of the interferent.

In a further aspect of the present disclosure, the second bias voltage value may be lower than the first bias voltage value.

In yet a further aspect of the present disclosure, the method may further include: determining at least one of a second EIS parameter value or a second conductivity value in response to exposure of the electrode to the analyte; and determining the interferent being below a threshold amount based on at least one of the second EIS parameter value or the second conductivity value.

In an aspect of the present disclosure, the method may further include adjusting the bias voltage of the working electrode in response to the interferent being below the threshold amount.

In yet a further aspect of the present disclosure, adjusting the bias voltage of the working electrode in response to the interferent being below the threshold amount may include ramping down the bias voltage.

In an aspect of the present disclosure, the bias voltage may be within a range of insulin excipient oxidation.

In yet another aspect of the present disclosure, determining that the analyte value is over the threshold may be based on obtaining a size value of the bolus from the pump.

Described but not claimed is that the method may further include, prior to the delivery of the bolus: obtaining the signal from the working electrode indicating an analyte value; determining that the analyte value is over a predetermined threshold; and causing the pump to deliver the bolus in response to the analyte value being over the predetermined threshold.

In accordance with aspects of the disclosure, one or more non-transitory processor-readable media store instructions which, when executed by one or more processors, cause performance at least of: obtaining an indication from a pump that a bolus is delivered; in response to the delivery of the bolus, determining at least one of a first electrochemical impedance spectroscopy (EIS) parameter value or a first conductivity value in response to exposure of a working electrode of an analyte sensor to an analyte; and determining a presence of an interferent based on at least one of the first EIS parameter value or the first conductivity value.

The details of one or more aspects of the disclosure are set forth in the accompanying drawings and the description below. Other features, objects, and advantages of the techniques described in this disclosure will be apparent from the description and drawings, and from the claims.

Further disclosed herein is an analyte sensor configured to compensate for insulin interference which includes: a working electrode, including an analyte sensing molecule disposed on the working electrode configured to generate a signal when exposed to an analyte; a processor; and a memory. The memory includes instructions which, when executed by the processor, cause the sensor to: obtain an indication from the pump that the bolus is delivered; in response to the delivery of the bolus, determine at least one of a first electrochemical impedance spectroscopy (EIS) parameter value or a first conductivity value in response to exposure of the working electrode to the analyte; and determine a presence of one or more interferents based on at least one of the first EIS parameter value or the first conductivity value.

### BRIEF DESCRIPTION OF THE DRAWINGS

A detailed description of aspects of the disclosure will be made with reference to the accompanying drawings, wherein like numerals designate corresponding parts in the figures.
FIG. 1 illustrates a perspective view of a subcutaneous sensor insertion set and a block diagram of an analyte sensor electronics device, in accordance with one or more aspects;
FIG. 2 illustrates a substrate having a first side which contains an electrode configuration and a second side which contains electronic circuitry, in accordance with one or more aspects;
FIG. 3 illustrates a block diagram of an electronic circuit for sensing an output of an analyte sensor, in accordance with one or more aspects;
FIG. 4 illustrates a block diagram of an analyte sensor electronics device and a sensor including a plurality of electrodes, in accordance with one or more aspects;
FIG. 5 illustrates an alternative aspect, including a sensor and a sensor electronics device, in accordance with one or more aspects;
FIG. 6 is a flow diagram for a method for compensating for insulin interference of an analyte sensor, in accordance with aspects of the disclosure;
FIG. 7 is a graph illustrating the polymerization of phenol on bare platinum, in accordance with aspects of the disclosure;
FIG. 8 is a graph illustrating ferricyanide (FeCN) cyclic voltammetry (CV) to verify phenol film formation, in accordance with aspects of the disclosure;
FIG. 9 is a graph illustrating Ferricyanide CV characterization of the effect of applied bias voltage on phenol poisoning of bare platinum, in accordance with one or more aspects of the disclosure;
FIG. 10 is a graph illustrating a first example bias voltage, in accordance with one or more aspects of the disclosure;
FIG. 11 is a graph illustrating a second example bias voltage, in accordance with one or more aspects of the disclosure;
FIG. 12 is a graph illustrating a third example bias voltage, in accordance with one or more aspects of the disclosure;
FIG. 13 is a graph illustrating a fourth example bias voltage, in accordance with one or more aspects of the disclosure;
FIG. 14 is a graph illustrating a fifth example bias voltage, in accordance with one or more aspects of the disclosure;
FIG. 15 is a diagram illustrating an example insulin excipient's concentration profile, in accordance with one or more aspects of the disclosure;
FIGS. 16A and 16B are a model for illustrating a subcutaneous injection insulin depot for a 5U bolus, in accordance with one or more aspects of the disclosure;
FIGS. 17A and 17B are a model for illustrating a subcutaneous injection insulin depot for a 10U bolus, in accordance with one or more aspects of the disclosure;
FIG. 18 is a table illustrating a working electrode depth and time in a phenol concentration, in accordance with one or more aspects of the disclosure;
FIG. 19A-19C are computed tomography (CT) images of various boluses in tissue, in accordance with one or more aspects of the disclosure; and
FIG. 20 is a diagram illustrating the use of conductivity and/or electrochemical impedance spectroscopy (EIS) to adjust the bias voltage for the sensor of FIG. 1, in accordance with one or more aspects of the disclosure.

### DETAILED DESCRIPTION

In the following description, reference is made to the accompanying drawings which form a part hereof and which illustrate several aspects of the present disclosure. It is understood that other aspects may be utilized, and structural and operational changes may be made without departing from the scope of the present disclosure.

The aspects herein are described below with reference to flowchart illustrations of methods, systems, devices, apparatus, processor-executable products, processor-executable instructions, and programming and computer program products. It will be understood that each block of the flowchart illustrations, and combinations of blocks in the flowchart illustrations, can be implemented by programming instructions, including computer program instructions (as can any menu screens described in the figures). These computer program instructions may be loaded onto a computer or other programmable data processing apparatus (such as a controller, microcontroller, or processor in a sensor electronics device) to produce a machine, such that the instructions which execute on the computer or other programmable data processing apparatus create instructions for implementing the functions specified in the flowchart block or blocks. These computer program instructions may also be stored in a computer-readable memory that can direct a computer or other programmable data processing apparatus to function in a particular manner, such that the instructions stored in the computer-readable memory produce an article of manufacture including instructions which implement the function specified in the flowchart block or blocks. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions specified in the flowchart block or blocks, and/or menus presented herein. Programming instructions may also be stored in and/or implemented via electronic circuitry, including integrated circuits (ICs) and Application Specific Integrated Circuits (ASICs) used in conjunction with sensor devices, apparatuses, and systems.

In diabetes therapy, it is known that components of insulin may interact with the glucose sensor when infused in proximity. These interactions may interfere with the sensitivity of the sensor. The disclosed technology provides the benefit of adjusting the bias voltage for the sensor's electrodes for a more accurate reading based on utilizing communication between the insulin pump and the sensor.

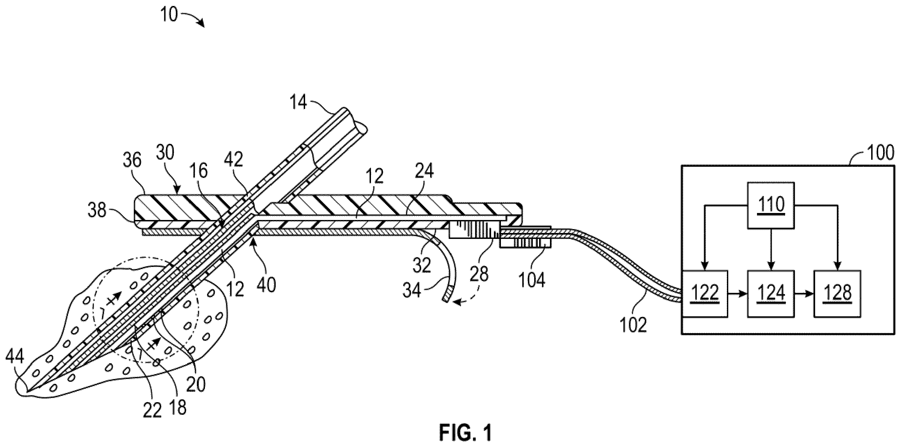

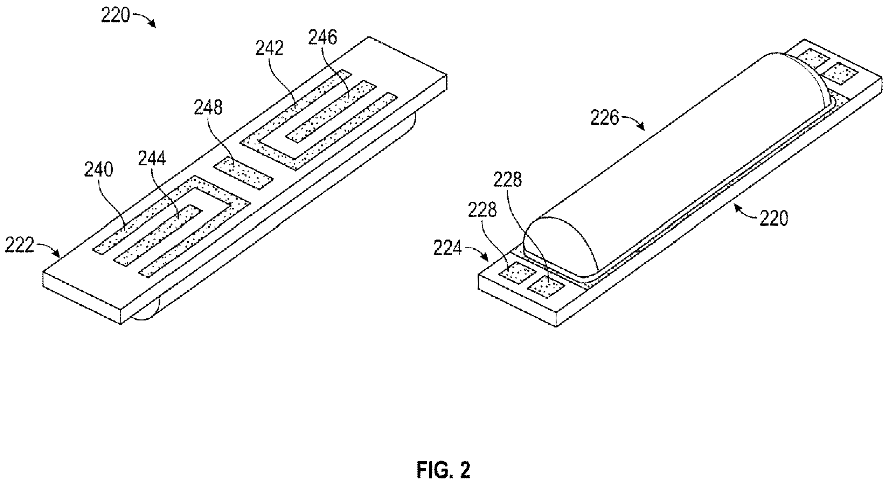

FIG. 1 is a perspective view of a subcutaneous sensor insertion set and a block diagram of a sensor electronics device according to various aspects of the disclosure. As illustrated in FIG. 1, a subcutaneous sensor set 10 is provided for subcutaneous placement of an active portion of an analyte sensor 12 (see, e.g., FIG. 2), or the like, at a selected site in the body of a user. The subcutaneous or percutaneous portion of the sensor set 10 includes a hollow, slotted insertion needle 14, and a cannula 16. The needle 14 is used to facilitate quick and easy subcutaneous placement of the cannula 16 at the subcutaneous insertion site. Inside the cannula 16 is a sensing portion 18 of the analyte sensor 12 to expose one or more sensor electrodes 20 to the user's bodily fluids through a window 22 formed in the cannula 16. In an aspect of the disclosure, the one or more sensor electrodes 20 may include a counter electrode, a reference electrode, and one or more working electrodes. After insertion, the insertion needle 14 is withdrawn to leave the cannula 16 with the sensing portion 18 and the sensor electrodes 20 in place at the selected insertion site.

In particular aspects, the subcutaneous sensor set 10 facilitates accurate placement of a flexible thin film electrochemical analyte sensor 12 of the type used for monitoring specific blood parameters representative of a user's condition. The analyte sensor 12 monitors glucose levels in the body and may be used in conjunction with automated or semi-automated medication infusion pumps of the external or implantable type as described, e.g., in U.S. Patent Nos. 4,562,751; 4,678,408; 4,685,903; or 4,573,994, to control delivery of insulin to a diabetic patient.

Particular aspects of the flexible analyte sensor 12 are constructed in accordance with thin film mask techniques to include elongated thin film conductors embedded or encased between layers of a selected insulative material such as polyimide film or sheet, and membranes. The sensor electrodes 20 at a tip end of the sensing portion 18 are exposed through one of the insulative layers for direct contact with patient blood or other body fluids, when the sensing portion 18 (or active portion) of the analyte sensor 12 is subcutaneously placed at an insertion site. The sensing portion 18 is joined to a connection portion 24 that terminates in conductive contact pads, or the like, which are also exposed through one of the insulative layers. In alternative aspects, other types of implantable sensors, such as chemical based, optical based, or the like, may be used.

As is known in the art, the connection portion 24 and the contact pads are generally adapted for a direct wired electrical connection to a suitable monitor or sensor electronics device 100 for monitoring a user's condition in response to signals derived from the sensor electrodes 20. Further description of flexible thin film sensors of this general type may be found, e.g., in U.S. Patent No. 5,391,250. The connection portion 24 may be conveniently connected electrically to the monitor or sensor electronics device 100 or by a connector block 28 (or the like) as shown and described, e.g., in U.S. Patent No. 5,482,473. Thus, in accordance with aspects of the present disclosure, subcutaneous sensor sets 10 may be configured or formed to work with either a wired or a wireless characteristic monitor system.

The sensor electrodes 20 may be used in a variety of sensing applications and may be configured in a variety of ways. For example, the sensor electrodes 20 may be used in physiological parameter sensing applications in which some type of biomolecule is used as a catalytic agent. For example, the sensor electrodes 20 may be used in an oxygen-independent glucose sensor.

The sensor electrodes 20, along with a biomolecule or some other catalytic agent, may be placed in a human body in a vascular or non-vascular environment. For example, the sensor electrodes 20 and biomolecule may be placed in a vein and be subjected to a blood stream, or may be placed in a subcutaneous or peritoneal region of the human body.

The monitor 100 may also be referred to as a sensor electronics device 100. The monitor 100 may include a power source 110, a sensor interface 122, processing electronics 124, and data formatting electronics 128. The monitor 100 may be coupled to the sensor set 10 by a cable 102 through a connector that is electrically coupled to the connector block 28 of the connection portion 24. In an alternative aspect, the cable 102 may be omitted. In this aspect of the disclosure, the monitor 100 may include an appropriate connector for direct connection to the connection portion 104 of the sensor set 10. The sensor set 10 may be modified to have the connector portion 104 positioned at a different location, e.g., on top of the sensor set 10 to facilitate placement of the monitor 100 over the sensor set 10.

In aspects of the disclosure, the sensor interface 122, the processing electronics 124, and the data formatting electronics 128 are formed as separate semiconductor chips, however, alternative aspects may combine the various semiconductor chips into a single or multiple customized semiconductor chips. The sensor interface 122 connects with the cable 102 that is connected with the sensor set 10.

The power source 110 may be a battery. The battery can include three series silver oxide battery cells. In alternative aspects, different battery chemistries may be utilized, such as lithium based chemistries, alkaline batteries, nickel metal hydride, or the like, and a different number of batteries may be used. The monitor 100 provides power to the sensor set via the power source 110, through the cable 102 and cable connector 104. In an aspect of the disclosure, the power is a voltage provided to the sensor set 10. In an aspect of the disclosure, the power is a current provided to the sensor set 10. In an aspect of the disclosure, the power is a voltage provided at a specific voltage to the sensor set 10.

FIG. 2 illustrates an implantable analyte sensor and electronics for driving the implantable analyte sensor according to an aspect of the present disclosure. FIG. 2 shows a substrate or flex 220 having two sides; a first side 222 which contains an electrode configuration and a second side 224 of which contains electronic circuitry. As in FIG. 2, the first side 222 of the substrate includes two counter electrode-working electrode pairs 240, 242, 244, 246 on opposite sides of a reference electrode 248. A second side 224 of the substrate includes electronic circuitry. As shown, the electronic circuitry may be enclosed in a hermetically sealed casing 226, providing a protective housing for the electronic circuitry. This allows the sensor substrate 220 to be inserted into a vascular environment or other environment which may subject the electronic circuitry to fluids. By sealing the electronic circuitry in a hermetically sealed casing 226, the electronic circuitry may operate without risk of short-circuiting by the surrounding fluids. Also shown in FIG. 2, pads 228 are connected to the input and output lines of the electronic circuitry. The electronic circuitry itself may be fabricated in a variety of ways. According to an aspect of the present disclosure, the electronic circuitry may be fabricated as an integrated circuit using techniques common in the industry.

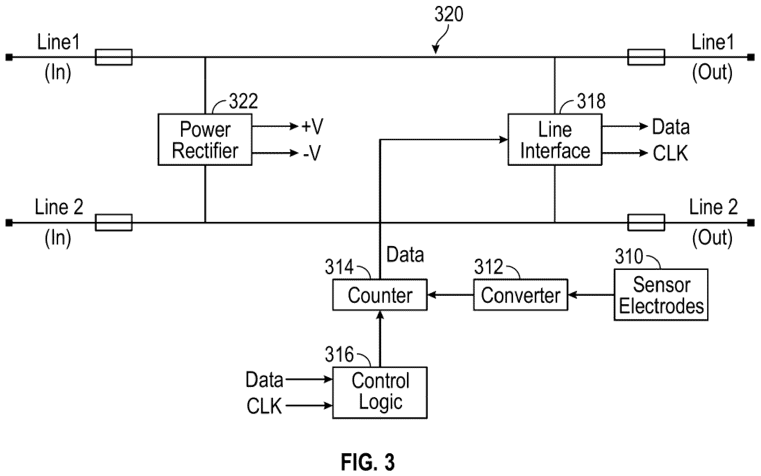

FIG. 3 illustrates a general block diagram of an electronic circuit for sensing the output of an analyte sensor according to aspects of the present disclosure. At least one pair of sensor electrodes 310 may interface to a data converter 312, the output of which may interface to a counter 314. The counter 314 may be controlled by control logic 316. The output of the counter 314 may connect to a line interface 318. The line interface 318 may be connected to input and output lines 320 and may also connect to the control logic 316. The input and output lines 320 may also be connected to a power rectifier 322.

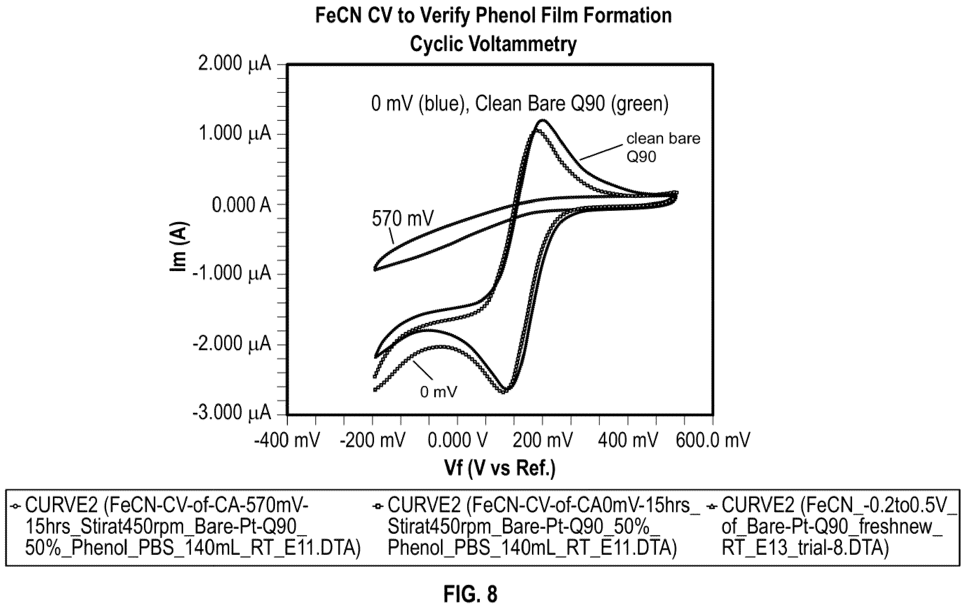

The sensor electrodes 310 may be used in a variety of sensing applications and may be configured in a variety of ways. For example, the sensor electrodes 310 may be used in physiological parameter sensing applications in which some type of biomolecule is used as a catalytic agent. For example, the sensor electrodes 310 may be used in an oxygen-independent glucose sensor having Boronic acid viologen (oBBV) that binds with the glucose. The sensor electrodes 310, along with oBBV and/or a dye (FIG. 8), may be placed in a human body in a vascular or non-vascular environment. For example, the sensor electrodes 310 and biomolecule may be placed in a vein and be subjected to a blood stream.

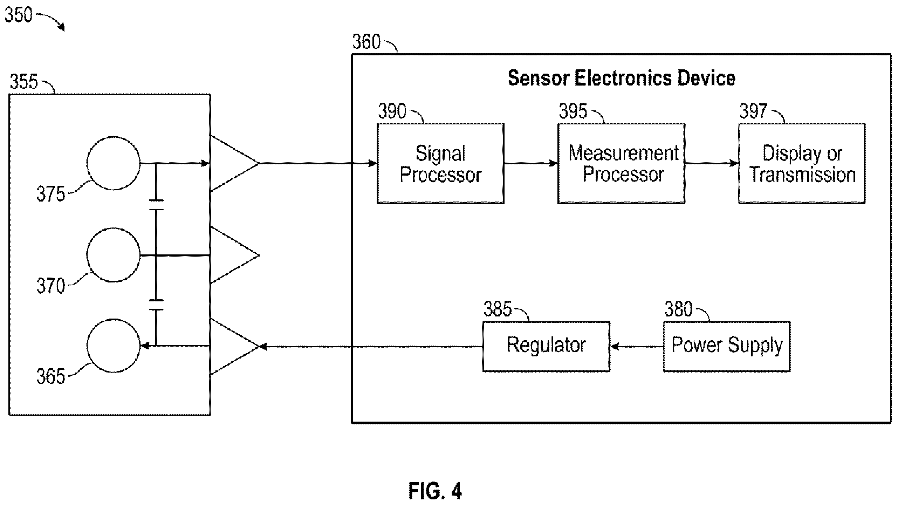

FIG. 4 illustrates a block diagram of an analyte sensor electronics device and a sensor including a plurality of electrodes according to an aspect of the disclosure. The sensor set or system 350 includes an analyte sensor 355 and a sensor electronics device 360. The analyte sensor 355 includes a counter electrode 365, a reference electrode 370, and a working electrode 375. The sensor electronics device 360 includes a power supply 380, a regulator 385, a signal processor 390, a measurement processor 395, and a display/transmission module 397. The power supply 380 provides power (in the form of either a voltage, a current, or a voltage including a current) to the regulator 385. The regulator 385 transmits a regulated voltage to the analyte sensor 355. In an aspect of the disclosure, the regulator 385 transmits a voltage to the counter electrode 365 of the analyte sensor 355.

The analyte sensor 355 creates a sensor signal indicative of a concentration of a physiological characteristic being measured. For example, the sensor signal may be indicative of a blood glucose reading. The sensor signal may be measured at the working electrode 375. In an aspect of the disclosure, the sensor signal may be a current measured at the working electrode. In an aspect of the disclosure, the sensor signal may be a voltage measured at the working electrode.

The signal processor 390 receives the sensor signal (e.g., a measured current or voltage) after the sensor signal is measured at the analyte sensor 355 (e.g., the working electrode). The signal processor 390 processes the sensor signal and generates a processed sensor signal. The measurement processor 395 receives the processed sensor signal and calibrates the processed sensor signal utilizing reference values. In an aspect of the disclosure, the reference values are stored in a reference memory and provided to the measurement processor 395. The measurement processor 395 generates sensor measurements. The sensor measurements may be stored in a measurement memory (not shown). The sensor measurements may be sent to a display/transmission device to be either displayed on a display in a housing with the sensor electronics or transmitted to an external device.

The sensor electronics device 360 may be a monitor that includes a display to display physiological characteristics readings. The sensor electronics device 360 may also be installed in a desktop computer, a pager, a television including communications capabilities, a laptop computer, a server, a network computer, a personal digital assistant (PDA), a portable telephone including computer functions, an infusion pump including a display, and/or a combination infusion pump/ analyte sensor. The sensor electronics device 360 may be housed in a cellular phone, a smartphone, a network device, a home network device, and/or other appliances connected to a home network.

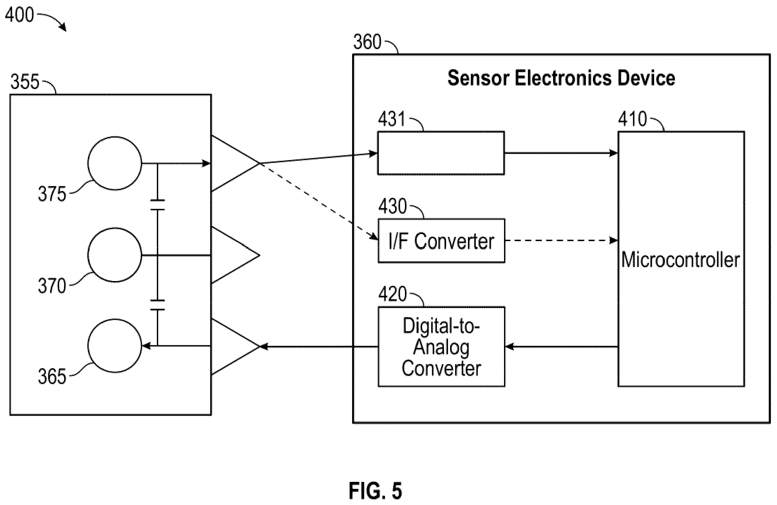

FIG. 5 illustrates an alternative aspect, including an analyte sensor and a sensor electronics device according to an aspect of the present disclosure. The sensor set or sensor system 400 includes the sensor electronics device 360 and the analyte sensor 355. The analyte sensor 355 includes the counter electrode 365, the reference electrode 370, and the working electrode 375. The sensor electronics device 360 includes a microcontroller 410 and a digital-to-analog converter (DAC) 420. The sensor electronics device 360 may also include a current-to-frequency converter (I/F converter) 430.

The microcontroller 410 includes software program code or programmable logic which, when executed, causes the microcontroller 410 to transmit a signal to the DAC 420, where the signal is representative of a voltage level or value that is to be applied to the analyte sensor 355. The DAC 420 receives the signal and generates the voltage value at the level instructed by the microcontroller 410. In aspects of the disclosure, the microcontroller 410 may change the representation of the voltage level in the signal frequently or infrequently. Illustratively, the signal from the microcontroller 410 may instruct the DAC 420 to apply a first voltage value for one second and a second voltage value for two seconds.

The analyte sensor 355 may receive the voltage level or value. In an aspect of the disclosure, the counter electrode 365 may receive the output of an operational amplifier which has as inputs the reference voltage and the voltage value from the DAC 420. The application of the voltage level causes the analyte sensor 355 to create a sensor signal indicative of a concentration of a physiological characteristic being measured. In an aspect of the disclosure, the microcontroller 410 may measure the sensor signal (e.g., a current value) from the working electrode. Illustratively, a sensor signal measurement circuit 431 may measure the sensor signal. In an aspect of the disclosure, the sensor signal measurement circuit 431 may include a resistor and the current may be passed through the resistor to measure the value of the sensor signal. In an aspect of the disclosure, the sensor signal may be a current level signal and the sensor signal measurement circuit 431 may be a current-to-frequency (I/F) converter 430. The I/F converter 430 may measure the sensor signal in terms of a current reading, convert it to a frequency-based sensor signal or electrochemical impedance spectroscopy ("EIS") signal, and transmit the frequency-based sensor signal or EIS signal to the microcontroller 410. Persons skilled in the art will understand how to implement and apply EIS. Various aspects of EIS signals are described in U.S. Patent Application Publication No. US2013/0060105A1. In aspects of the disclosure, the microcontroller 410 may be able to receive frequency-based sensor signals easier than non-frequency-based sensor signals. The microcontroller 410 receives the sensor signal, whether frequency-based or non-frequency-based, and determines a value for the physiological characteristic of a subject, such as a blood glucose level. The microcontroller 410 may include program code, which when executed or run, is able to receive the sensor signal and convert the sensor signal to a physiological characteristic value.

In one aspect of the disclosure, the microcontroller 410 may convert the sensor signal to a blood glucose level. While converting the sensor signal to a blood glucose value, the microcontroller 410 may use one or more models, which are specific ways to use the sensor signal to calculate the blood glucose value. In some aspects, the microcontroller 410 may utilize measurements (e.g., sensor signals and electrochemical impedance spectroscopy (EIS) signals from the analyte sensor 355) stored within an internal memory in order to determine the blood glucose level of the subject. In some aspects, the microcontroller 410 may utilize measurements stored within a memory external to the microcontroller 410 to assist in determining the blood glucose level of the subject.

After the physiological characteristic value is determined by the microcontroller 410, the microcontroller 410 may store measurements of the physiological characteristic values for a number of time periods. For example, a blood glucose value (BG) may be sent to the microcontroller 410 from the sensor every second or five seconds, and the microcontroller may save sensor measurements for five minutes or ten minutes of BG readings. The microcontroller 410 may transfer the measurements of the physiological characteristic values to a display on the sensor electronics device 360. For example, the sensor electronics device 360 may be a monitor which includes a display that provides a blood glucose reading for a subject. In one aspect of the disclosure, the microcontroller 410 may transfer the measurements of the physiological characteristic values to an output interface of the microcontroller 410. The output interface of the microcontroller 410 may transfer the measurements of the physiological characteristic values, e.g., blood glucose values, to an external device, e.g., an infusion pump 530, a combined infusion pump/glucose meter, a computer, a personal digital assistant, a pager, a network appliance, a server, a cellular phone, or any computing device.

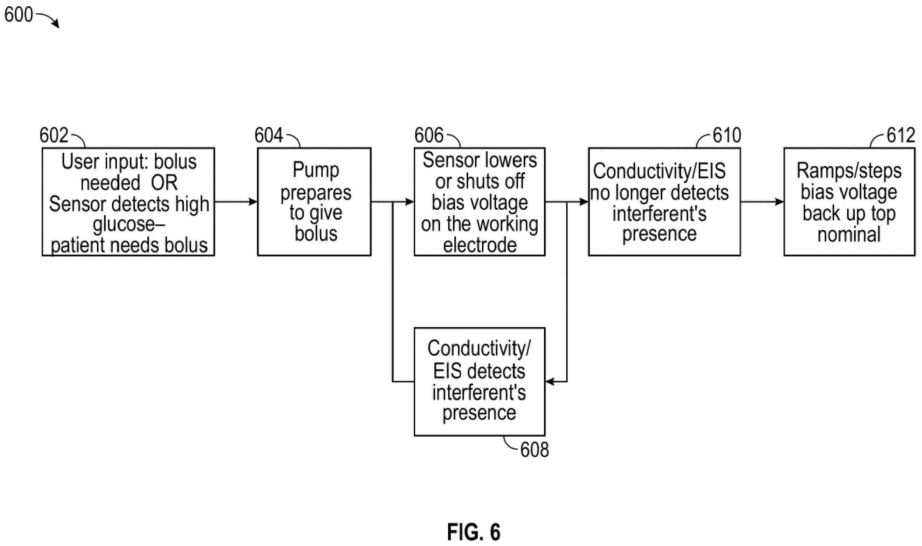

FIG. 6 is a flow diagram for processor-implemented method 600 of compensating for insulin interference for the analyte sensor 12 of FIG. 1. The method 600 may be implemented by sensor electronics device 100 of FIG. 1 or sensor electronics device 360 of FIGS. 4 and 5.

Generally, the analyte sensor applies a bias voltage to the working electrode to adjust the sensitivity of a measurement of an analyte. The bias voltage is within a range of insulin excipient oxidation (e.g., 300-600mV). This can cause the delivery of a bolus (e.g., insulin) to interfere with the accuracy of the sensor's measurements.

At block 602, the processor causes the analyte sensor 12 to obtain an indication that a bolus will be delivered (e.g., from a pump 530 (FIG. 5) in communication with the sensor 12). For example, prior to the delivery of the bolus, the processor may cause the analyte sensor to obtain the signal from the working electrode indicating an analyte value, determine that the analyte value is over the predetermined threshold, and cause a pump 530 to deliver the bolus in response to the analyte value being over the predetermined threshold. In aspects, the analyte sensor may obtain the size of the bolus from the pump. For example, the pump 530 may transmit to the sensor the size of the bolus to be delivered at a specific point in time (e.g., 5U).

In another example, prior to the delivery of the bolus, the processor may cause the analyte sensor to obtain input from a user to cause the pump to deliver the bolus and cause the pump to deliver the bolus in response to the input. The input may include the size of the bolus.

At block 604, the pump 530 prepares to deliver the bolus at a point in time. In aspects, the processor may cause the sensor to obtain an indication from the pump that the bolus is delivered at a specific point in time.

In aspects, the processor may cause the sensor, in response to the delivery of the bolus, to obtain the signal from the working electrode. The signal may be processed to generate an electrochemical impedance spectroscopy (EIS) parameter value and/or a conductivity value.

At block 606, if the analyte value is over a predetermined threshold, then the processor causes the sensor 12 to reduce or shut off the bias voltage on the working electrode. The operation of block 606 will be described in more detail below herein.

At block 608, the processor causes the sensor 12 to generate an electrochemical impedance spectroscopy (EIS) parameter value and/or a conductivity value in response to exposure of the working electrode to the analyte. In aspects, the processor determines the presence of an interferent (e.g., insulin) based on at least one of the first EIS parameter values or the first conductivity value. An interferent is a substance that interferes with an analytical procedure and produces incorrect results.

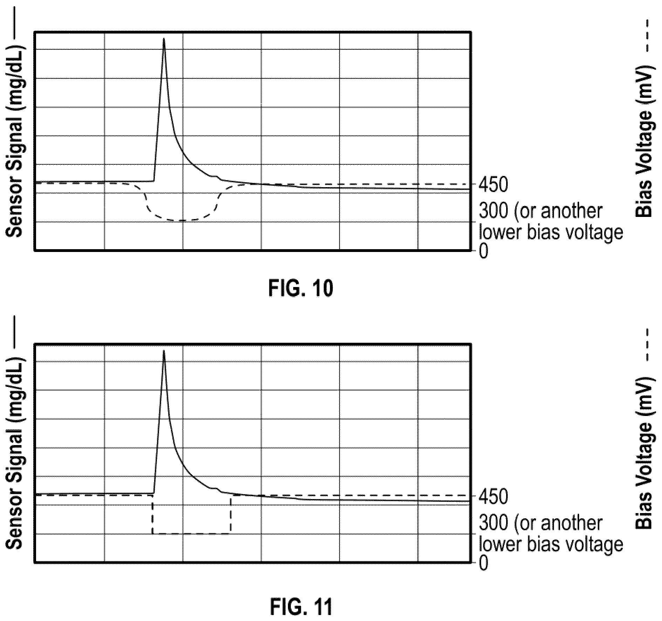

Blocks 606 and 608 are iteratively performed. For example, if the EIS or conductivity measurements still indicate the presence of the interferent, then the processor causes the sensor to lower the bias voltage of the working electrode. Thus, the sensor may adjust the bias voltage of the working electrode from a first bias voltage value to a second bias voltage value in response to the presence of the interferent. The second bias voltage value is lower than the first bias voltage value. For example, the bias voltage of the working electrode may be adjusted by ramping down the bias voltage or stepping down to a lower value. Further aspects of this adjustment will be described in connection with FIGS. 10-14.

At block 610, the sensor 12 no longer detects the presence of the interferent. For example, time has passed, and the insulin has dropped to a low enough value that it does not interfere with the accuracy of the sensor 12 signal's measurements. For example, the sensor 12 may determine a second EIS parameter value and/or a second conductivity value in response to exposure of the electrode to the analyte. The sensor 12 may determine an absence of the interferents, or that the interferents are below a threshold amount, based on at least one of the second EIS parameter values or the second conductivity value. In aspects, the processor may cause the sensor 12 to adjust the bias voltage of the working electrode in response to the absence of the interferent. For example, the processor may remeasure the EIS parameter value to test for the presence or absence of insulin. The sensor 12 may determine there is no insulin based on the EIS parameter value. For example, the threshold amount may be below an amount that causes the working electrode to lose measuring sensitivity and/or accuracy of more than about 1%.

In aspects, the sensor 12, may provide one or more shapes/curves for the adjusted bias voltage, which will be described below in connection with FIGS. 10-14. For example, a user interface may display the one or more curves. In another aspect, the one or more curves may be selected by the sensor 12 based on at least one of a type or model of sensor 12 or a bolus dosage indicated by the pump 530.

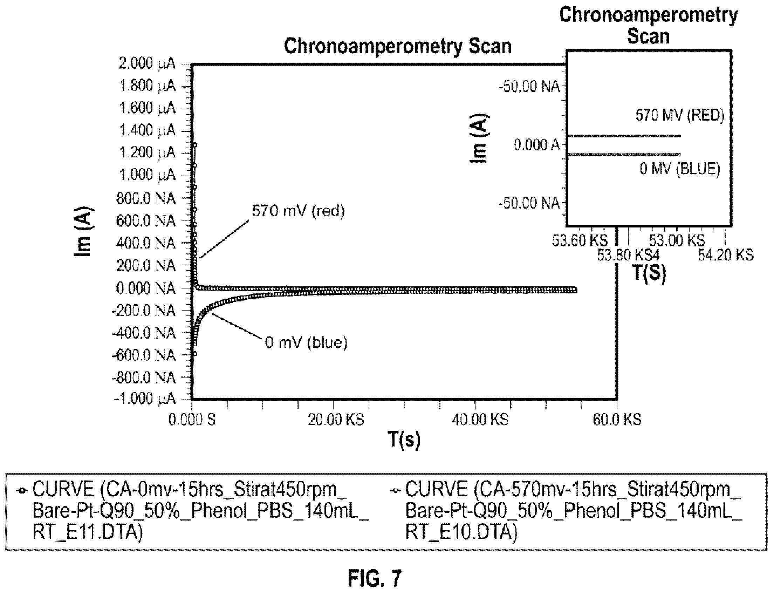

Regarding FIGS. 7 and 8, graphs illustrating current vs time (FIG. 7) and voltage vs time (FIG. 8) for an electro-polymerization of phenol on bare platinum electrodes (Pt) of the sensor of FIG. 1 using the chronoamperometry method are shown. An 8mM phenol in PBS (phosphate buffered saline) is prepared at 37 C, stirring at about 200 rpm, using an electro-polymerization method of chronoamperometry for about 15 hours. PBS was used as an example indicator. The disappearance of the red-ox peaks on the FeCN cyclic voltammetry (CV) curve verifies the blocking behavior of phenol on the platinum electrode at a bias potential of 570 mV. The FeCN CV red-ox peaks appear the same for a clean platinum electrode, and the platinum electrode was dipped in a phenol solution where 0 mV was applied, confirming that phenol poisoning does not occur at 0 mV. Here, phenol is used as an example instead of insulin.

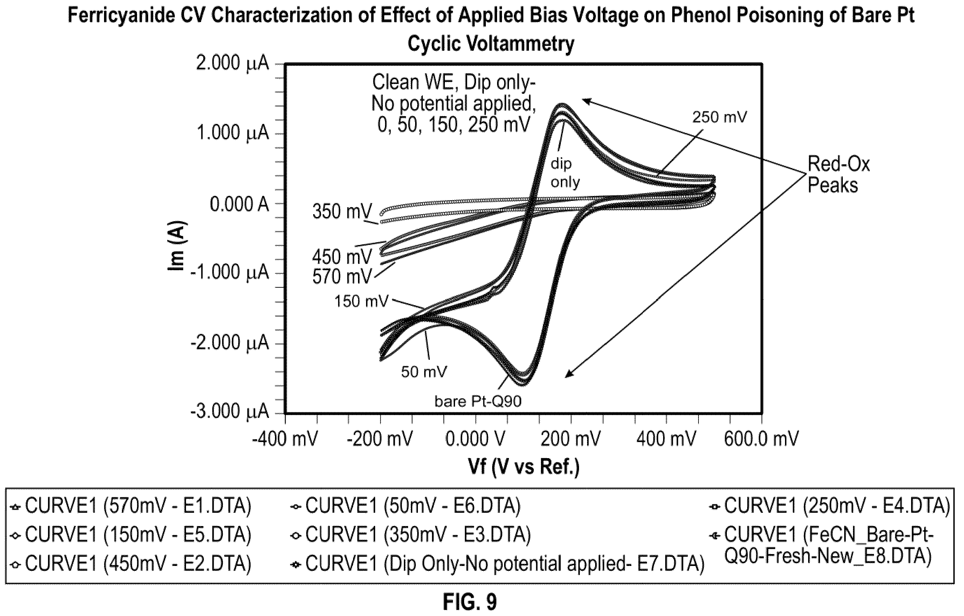

FIG. 9 is a graph illustrating current vs voltage illustrating ferricyanide CV characterization of the effect of applied bias voltage on phenol poisoning of bare platinum for the sensor of FIG. 1. An 8mM of phenol in PBS at about 37 C is used, with stirring at about 200 rpm, using an electro-polymerization method of chronoamperometry for about 2 hours. Below about 300 mV, red-ox peaks can be seen for the working electrode.

FIG. 10 is a graph illustrating a first example bias voltage. The bias voltage ramps down to a lower voltage during the time period where interference occurs. As the sensor signal returns to normal, the bias voltage is configured to slowly ramp back to normal.

FIG. 11 is a graph illustrating a second example bias voltage. The bias voltage steps down to lower voltage during the time period where interference occurs. As sensor signal returns to normal, the bias voltage is configured to step back to normal.

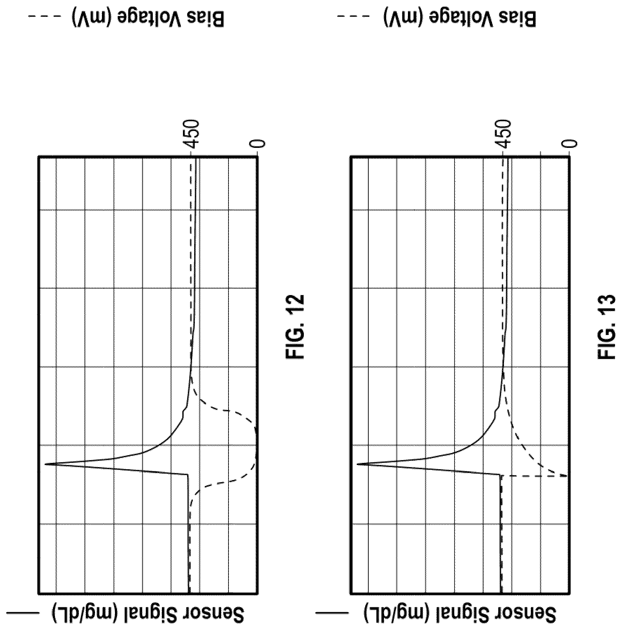

FIG. 12 is a graph illustrating a third example bias voltage. The bias voltage ramps down to 0mV during the time period where interference occurs. As the sensor signal returns to normal, the bias voltage is configured to slow ramps back to normal.

FIG. 13 is a graph illustrating a fourth example bias voltage. The bias voltage steps down to 0mV during the time period when interference occurs. As the sensor signal returns to normal, the bias voltage is configured to slowly ramp back to normal.

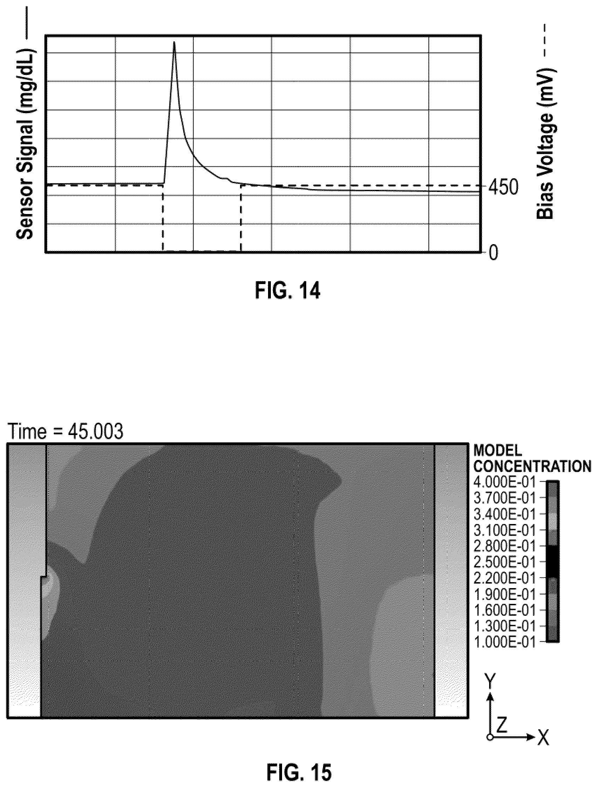

FIG. 14 is a graph illustrating a fifth example bias voltage. The bias voltage steps down to 0mV during the time period where interference occurs. As the sensor signal returns to normal, the bias voltage is configured to step back to normal.

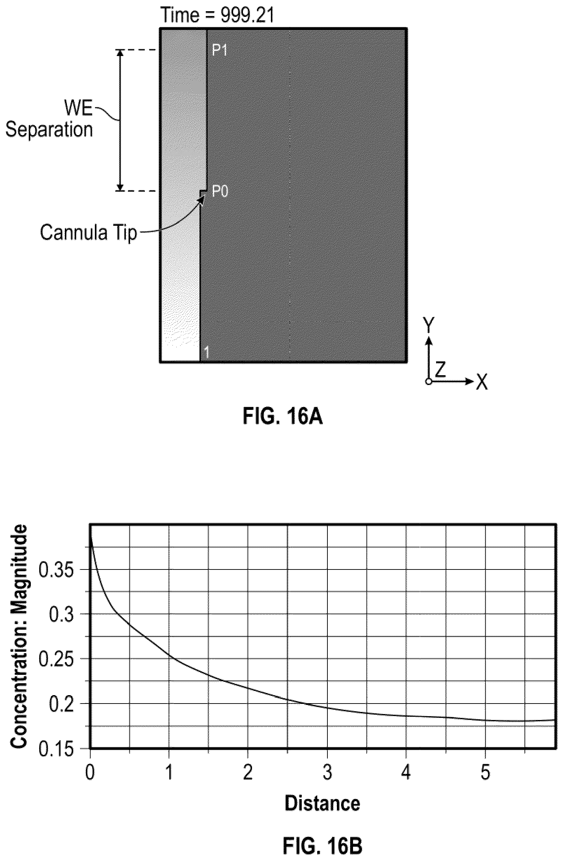

FIG. 15 illustrates a computational fluid dynamics (CFD) model of an example insulin excipient's concentration profile. FIGS. 16A and 16B illustrate a CFD model. The graph on FIG. 16B shows the insulin excipient concentration decays as a function of working electrode distance from the cannula tip for a 5U bolus. The x axis is distance and the Y axis is the magnitude of the concentration (FIG. 16B).

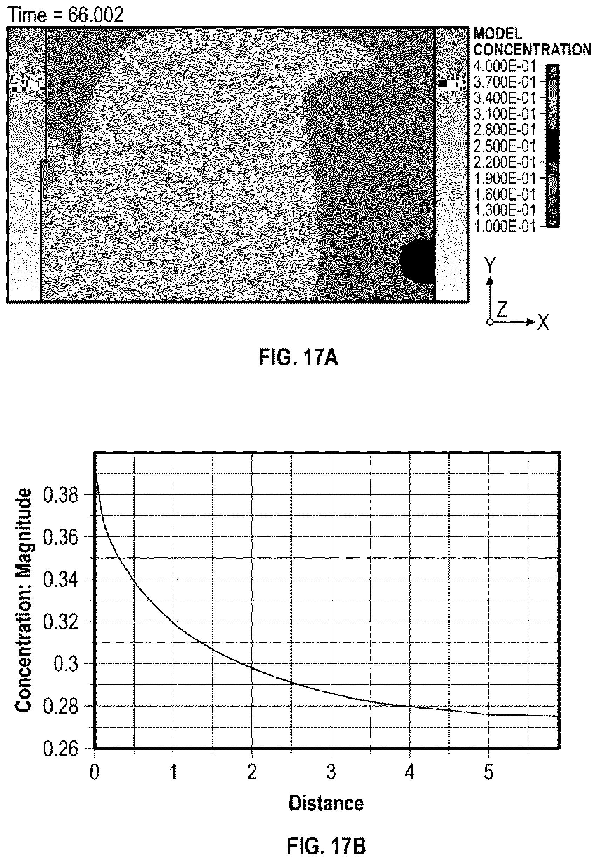

FIGS. 17A and 17B are a model for illustrating a subcutaneous injection insulin depot for a 10U bolus. Insulin excipient concentration decay as a function of working electrode distance from cannula tip for a 10U bolus. The x axis is distance and the Y axis is the magnitude of the concentration (FIG. 17B).

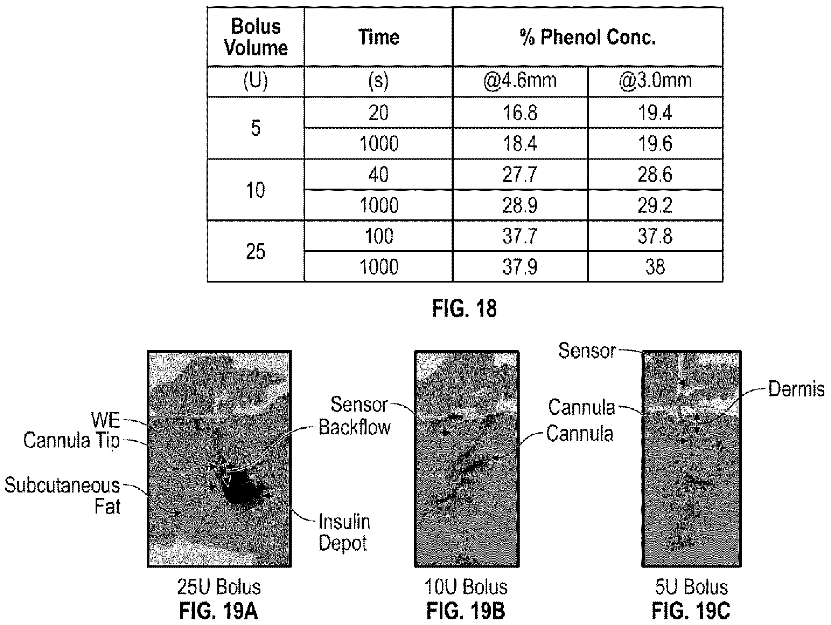

FIG. 18 is a table illustrating a working electrode depth and time in a phenol concentration. The percent phenol concentration for bolus volumes of 5U, 10U and 25U after different periods of time is shown.

FIG. 19A-19C are CT images of various boluses in tissue. FIG. 19A shows a 25U bolus in tissue. The working electrode of sensor 12 (FIG. 1) and the cannula tip can be seen in the insulin deposit in the tissue. FIG. 19B illustrates a 10U bolus, showing a significantly smaller insulin deposit in the tissue. FIG. 19C illustrates a 5U bolus in tissue.

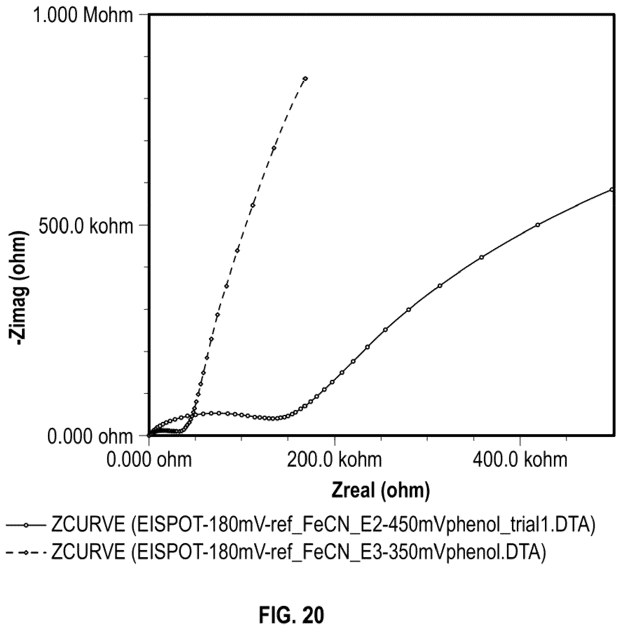

FIG. 20 is a diagram illustrating the use of conductivity and/or EIS to adjust the bias voltage for the sensor of FIG. 1.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, the above-described servers and computing devices.

While the description above refers to particular aspects of the present disclosure, it will be understood that many modifications may be made without departing from the spirit thereof. Additional steps and changes to the order of the algorithms can be made while still performing the key teachings of the present disclosure. Thus, the accompanying claims are intended to cover such modifications as would fall within the true scope of the present disclosure. The presently disclosed aspects are, therefore, to be considered in all respects as illustrative and not restrictive, the scope of the disclosure being indicated by the appended claims rather than the foregoing description. Unless the context indicates otherwise, any aspect disclosed herein may be combined with any other aspect or aspects disclosed herein. All changes that come within the meaning of, and range of, equivalency of the claims are intended to be embraced therein.

## Claims

1. An analyte sensor (355) configured to compensate for insulin interference, the analyte sensor comprising:
a working electrode (375) including an analyte sensing molecule disposed on the working electrode configured to generate a signal when exposed to an analyte, wherein the working electrode is biased at a first bias voltage value;
a processor; and
a memory, including instructions which, when executed by the processor, cause the sensor to:
obtain an indication from a pump (530) that a bolus is delivered;
in response to delivery of the bolus, determine at least one of a first electrochemical impedance spectroscopy (EIS) parameter value or a first conductivity value in response to exposure of the working electrode to the analyte; and
determine whether an interferent is present based on at least one of the first EIS parameter value or the first conductivity value.

2. The analyte sensor of claim 1, wherein the instructions, when executed by the processor, further cause the analyte sensor to:
adjust the bias voltage of the working electrode from the first bias voltage value to a second bias voltage value in response to determining the presence of the interferent, particularly wherein the second bias voltage value is lower than the first bias voltage value.

3. The analyte sensor of claim 1 or 2, wherein the instructions, when executed by the processor, further cause the analyte sensor to:
determine at least one of a second EIS parameter value or a second conductivity value in response to exposure of the electrode to the analyte; and
determine the interferent is present at an amount below a threshold amount based on at least one of the second EIS parameter value or the second conductivity value.

4. The analyte sensor of claim 3, wherein the instructions, when executed by the processor, further cause the analyte sensor to:
adjust the bias voltage of the working electrode in response to determining that the interferent is present in an amount below the threshold amount, particularly wherein the adjusting of the bias voltage of the working electrode in response to the interferent being below the threshold amount includes ramping down the bias voltage.

5. The analyte sensor of one of claims 1 to 4, wherein the first bias voltage is within a range of insulin excipient oxidation.

6. The analyte sensor of one of claims 1 to 5, wherein determining that the analyte value is over the threshold is based on obtaining a size value of the bolus from the pump.

7. The analyte sensor of one of claims 1 to 6, wherein the instructions, when executed by the processor, further cause the analyte sensor to:
prior to the delivery of the bolus:
obtain the signal from the working electrode indicating an analyte value;
determine that the analyte value is over a predetermined threshold; and
cause the pump to deliver the bolus in response to the analyte value being over the predetermined threshold.

8. The analyte sensor of one of claims 1 to 7, wherein the instructions, when executed by the processor, further cause the analyte sensor to:
prior to the delivery of the bolus:
obtain an input from a user to cause the pump to deliver the bolus, wherein the input includes a bolus size; and
cause the pump to deliver the bolus in response input.

9. A processor-implemented method of compensating for insulin interference for an analyte sensor (355), the method comprising:
obtaining an indication from a pump (530) that a bolus is delivered at a point in time;
in response to the delivery of the bolus, determining at least one of a first electrochemical impedance spectroscopy (EIS) parameter value or a first conductivity value in response to exposure of a working electrode (375) of an analyte sensor to an analyte; and
determining a presence of an interferent based on at least one of the first EIS parameter value or the first conductivity value.

10. The processor-implemented method of claim 9, further comprising:
adjusting a bias voltage of the working electrode from a first bias voltage value to a second bias voltage value in response to the presence of the interferent, particularly wherein the second bias voltage value is lower than the first bias voltage value.

11. The processor-implemented method of claim 9 or 10, further comprising:
determining at least one of a second EIS parameter value or a second conductivity value in response to exposure of the electrode to the analyte; and
determining the interferent being below a threshold amount based on at least one of the second EIS parameter value or the second conductivity value,
particularly further comprising:
adjusting the bias voltage of the working electrode in response to the interferent being below the threshold amount, particularly wherein the adjusting of the bias voltage of the working electrode in response to the interferent being below the threshold amount includes ramping down the bias voltage.

12. The processor-implemented method of one of claims 9 to 11, wherein the bias voltage is within a range of insulin excipient oxidation.

13. The processor-implemented method of one of claims 9 to 12, wherein the determining that the analyte value is over the threshold is based on obtaining a size value of the bolus from the pump.

14. One or more non-transitory processor-readable media storing instructions which, when executed by one or more processors of the analyte sensor (355) of one of claim 1 to 8, cause performance at least of:
obtaining an indication from a pump (530) that a bolus is delivered;
in response to the delivery of the bolus, determining at least one of a first electrochemical impedance spectroscopy (EIS) parameter value or a first conductivity value in response to exposure of a working electrode (375) of an analyte sensor to an analyte; and
determining a presence of an interferent based on at least one of the first EIS parameter value or the first conductivity value.

## Patentansprüche

1. Analytsensor (355), der konfiguriert ist, um Insulininterferenz auszugleichen, der Analytsensor umfassend:
eine Arbeitselektrode (375), die ein Analyterfassungsmolekül einschließt, das auf der Arbeitselektrode angeordnet ist, das konfiguriert ist, um ein Signal zu erzeugen, wenn es einem Analyten ausgesetzt wird, wobei die Arbeitselektrode mit einem ersten Vorspannungswert vorgespannt ist;
einen Prozessor; und
einen Speicher, der Anweisungen einschließt, die, wenn sie durch den Prozessor ausgeführt werden, den Sensor veranlassen zum:
Erhalten einer Angabe von einer Pumpe (530), dass ein Bolus abgegeben wird;
als Reaktion auf die Abgabe des Bolus, Bestimmen mindestens eines von einem ersten elektrochemischen Impedanzspektroskopieparamterwert (EIS-Parameterwert) oder einem ersten Leitfähigkeitswert als Reaktion auf das Aussetzen der Arbeitselektrode gegenüber dem Analyten; und
Bestimmen, ob eine Störsubstanz vorhanden ist, basierend auf mindestens einem des ersten EIS-Parameterwerts oder des ersten Leitfähigkeitswerts.

2. Analytsensor nach Anspruch 1, wobei die Anweisungen, wenn sie durch den Prozessor ausgeführt werden, den Analytsensor ferner veranlassen zum:
Einstellen der Vorspannung der Arbeitselektrode von dem ersten Vorspannungswert auf einen zweiten Vorspannungswert als Reaktion auf das Bestimmen des Vorhandenseins der Störsubstanz, insbesondere wobei der zweite Vorspannungswert niedriger als der erste Vorspannungswert ist.

3. Analytsensor nach Anspruch 1 oder 2, wobei die Anweisungen, wenn sie durch den Prozessor ausgeführt werden, den Analytsensor ferner veranlassen zum:
Bestimmen mindestens eines zweiten EIS-Parameterwerts oder eines zweiten Leitfähigkeitswerts als Reaktion auf das Aussetzen der Elektrode gegenüber dem Analyten; und
Bestimmen, dass die Störsubstanz in einer Menge unterhalb einer Schwellenmenge vorhanden ist, basierend auf mindestens einem von dem zweiten EIS-Parameterwert oder dem zweiten Leitfähigkeitswert.

4. Analytsensor nach Anspruch 3, wobei die Anweisungen, wenn sie durch den Prozessor ausgeführt werden, den Analytsensor ferner veranlassen zum:
Einstellen der Vorspannung der Arbeitselektrode als Reaktion auf das Bestimmen, dass die Störsubstanz in einer Menge unterhalb der Schwellenmenge vorhanden ist, insbesondere wobei das Einstellen der Vorspannung der Arbeitselektrode als Reaktion darauf, dass die Störsubstanz unterhalb der Schwellenmenge liegt, ein Absenken der Vorspannung einschließt.

5. Analytsensor nach einem der Ansprüche 1 bis 4, wobei die erste Vorspannung innerhalb eines Bereichs von Insulinhilfsstoffoxidation liegt.

6. Analytsensor nach einem der Ansprüche 1 bis 5, wobei das Bestimmen, dass der Analytwert über der Schwelle liegt, auf dem Erhalten eines Größenwerts des Bolus von der Pumpe basiert.

7. Analytsensor nach einem der Ansprüche 1 bis 6, wobei die Anweisungen, wenn sie durch den Prozessor ausgeführt werden, den Analytsensor ferner veranlassen zum:
vor der Abgabe des Bolus:
Erhalten des Signals von der Arbeitselektrode, das einen Analytwert angibt;
Bestimmen, dass der Analytwert über einer vorbestimmten Schwelle liegt;
und
Veranlassen, dass die Pumpe den Bolus als Reaktion darauf abgibt, dass der Analytwert über der vorbestimmten Schwelle liegt.

8. Analytsensor nach einem der Ansprüche 1 bis 7, wobei die Anweisungen, wenn sie durch den Prozessor ausgeführt werden, den Analytsensor ferner veranlassen zum:
vor der Abgabe des Bolus:
Erhalten einer Eingabe von einem Benutzer, um zu veranlassen, dass die Pumpe den Bolus abgibt, wobei die Eingabe eine Bolusgröße einschließt; und
Veranlassen, dass die Pumpe den Bolus als Reaktion auf eine Eingabe abgibt.

9. Prozessorimplementiertes Verfahren zum Ausgleichen von Insulininterferenz für einen Analytsensor (355), das Verfahren umfassend:
Erhalten einer Angabe von einer Pumpe (530), dass ein Bolus zu einem Zeitpunkt abgegeben wird;
als Reaktion auf die Abgabe des Bolus, Bestimmen mindestens eines von einem ersten elektrochemischen Impedanzspektroskopieparameterwert (EIS-Parameterwert) oder einem ersten Leitfähigkeitswert als Reaktion auf das Aussetzen einer Arbeitselektrode (375) eines Analytsensors gegenüber einem Analyten; und
Bestimmen eines Vorhandenseins einer Störsubstanz basierend auf mindestens einem von dem ersten EIS-Parameterwert oder dem ersten Leitfähigkeitswert.

10. Prozessorimplementiertes Verfahren nach Anspruch 9, ferner umfassend:
Einstellen einer Vorspannung der Arbeitselektrode von einem ersten Vorspannungswert auf einen zweiten Vorspannungswert als Reaktion auf das Vorhandensein der Störsubstanz, insbesondere wobei der zweite Vorspannungswert niedriger ist als der erste Vorspannungswert.

11. Prozessorimplementiertes Verfahren nach Anspruch 9 oder 10, ferner umfassend:
Bestimmen mindestens eines von einem zweiten EIS-Parameterwert oder einem zweiten Leitfähigkeitswert als Reaktion auf das Aussetzen der Elektrode gegenüber dem Analyten; und
Bestimmen, dass die Störsubstanz unter einer Schwellenmenge liegt, basierend auf mindestens einem des zweiten EIS-Parameterwerts oder des zweiten Leitfähigkeitswerts,
insbesondere ferner umfassend:
Einstellen der Vorspannung der Arbeitselektrode als Reaktion darauf, dass die Störsubstanz unter der Schwellenmenge liegt, insbesondere wobei das Einstellen der Vorspannung der Arbeitselektrode als Reaktion darauf, dass die Störsubstanz unter der Schwellenmenge liegt, ein Absenken der Vorspannung einschließt.

12. Prozessorimplementiertes Verfahren nach einem der Ansprüche 9 bis 11, wobei die Vorspannung innerhalb eines Bereichs von Insulinhilfsstoffoxidation liegt.

13. Prozessorimplementierte Verfahren nach einem der Ansprüche 9 bis 12, wobei das Bestimmen, dass der Analytwert über der Schwelle liegt, auf dem Erhalten eines Größenwerts des Bolus von der Pumpe basiert.

14. Ein oder mehrere nichtflüchtige prozessorlesbare Medien, die Anweisungen speichern, die, wenn sie durch einen oder mehrere Prozessoren des Analytsensors (355) nach einem der Ansprüche 1 bis 8 ausgeführt werden, die Durchführung veranlassen von mindestens:
Erhalten einer Angabe von einer Pumpe (530), dass ein Bolus abgegeben wird;
als Reaktion auf die Abgabe des Bolus, Bestimmen mindestens eines von einem ersten elektrochemischen Impedanzspektroskopieparameterwert (EIS-Parameterwert) oder einem ersten Leitfähigkeitswert als Reaktion auf das Aussetzen einer Arbeitselektrode (375) eines Analytsensors gegenüber einem Analyten; und
Bestimmen eines Vorhandenseins einer Störsubstanz basierend auf mindestens einem von dem ersten EIS-Parameterwert oder dem ersten Leitfähigkeitswert.

## Revendications

1. Capteur d'analyte (355) configuré pour compenser une interférence de l'insuline, le capteur d'analyte comprenant :
une électrode de travail (375) comportant une molécule de détection d'analyte disposée sur l'électrode de travail configurée pour générer un signal lorsqu'elle est exposée à un analyte, dans lequel l'électrode de travail est polarisée à une première valeur de tension de polarisation ;
un processeur ; et
une mémoire, comportant des instructions qui, lorsqu'elles sont exécutées par le processeur, amènent le capteur à :
obtenir une indication provenant d'une pompe (530) selon laquelle un bolus est administré ;
en réponse à l'administration du bolus, déterminer au moins l'une parmi une première valeur de paramètre de spectroscopie d'impédance électrochimique (EIS) ou une première valeur de conductivité en réponse à une exposition de l'électrode de travail à l'analyte ; et
déterminer si un interférent est présent en fonction d'au moins l'une parmi la première valeur de paramètre EIS ou la première valeur de conductivité.

2. Capteur d'analyte selon la revendication 1, dans lequel les instructions, lorsqu'elles sont exécutées par le processeur, amènent en outre le capteur d'analyte à :
ajuster la tension de polarisation de l'électrode de travail de la première valeur de tension de polarisation à une seconde valeur de tension de polarisation en réponse au fait de déterminer la présence de l'interférent, en particulier dans lequel la seconde valeur de tension de polarisation est inférieure à la première valeur de tension de polarisation.

3. Capteur d'analyte selon la revendication 1 ou 2, dans lequel les instructions, lorsqu'elles sont exécutées par le processeur, amènent en outre le capteur d'analyte à :
déterminer au moins l'une parmi une seconde valeur de paramètre EIS ou une seconde valeur de conductivité en réponse à une exposition de l'électrode à l'analyte ; et
déterminer que l'interférent est présent en une quantité inférieure à une quantité seuil en fonction d'au moins l'une parmi la seconde valeur de paramètre EIS ou la seconde valeur de conductivité.

4. Capteur d'analyte selon la revendication 3, dans lequel les instructions, lorsqu'elles sont exécutées par le processeur, amènent en outre le capteur d'analyte à :
ajuster la tension de polarisation de l'électrode de travail en réponse au fait de déterminer que l'interférent est présent en une quantité inférieure à la quantité seuil, en particulier dans lequel le fait d'ajuster la tension de polarisation de l'électrode de travail en réponse au fait que l'interférent est inférieur à la quantité seuil comporte la diminution progressive de la tension de polarisation.

5. Capteur d'analyte selon l'une des revendications 1 à 4, dans lequel la première tension de polarisation est comprise dans une plage d'oxydation d'excipient d'insuline.

6. Capteur d'analyte selon l'une des revendications 1 à 5, dans lequel le fait de déterminer que la valeur d'analyte est supérieure au seuil est basé sur l'obtention d'une valeur de taille du bolus à partir de la pompe.

7. Capteur d'analyte selon l'une des revendications 1 à 6, dans lequel les instructions, lorsqu'elles sont exécutées par le processeur, amènent en outre le capteur d'analyte à :
avant l'administration du bolus :
obtenir le signal provenant de l'électrode de travail et indiquant une valeur d'analyte ;
déterminer que la valeur d'analyte est supérieure à un seuil prédéterminé ; et
amener la pompe à administrer le bolus en réponse au fait que la valeur d'analyte est supérieure au seuil prédéterminé.

8. Capteur d'analyte selon l'une des revendications 1 à 7, dans lequel les instructions, lorsqu'elles sont exécutées par le processeur, amènent en outre le capteur d'analyte à :
avant l'administration du bolus :
obtenir une entrée provenant d'un utilisateur afin d'amener la pompe à administrer le bolus, dans lequel l'entrée comporte une taille de bolus ; et
amener la pompe à administrer le bolus en réponse à une entrée.

9. Procédé mis en œuvre par processeur pour la compensation d'une interférence de l'insuline pour un capteur d'analyte (355), le procédé comprenant :
l'obtention d'une indication provenant d'une pompe (530) selon laquelle un bolus est administré à un moment donné ;
en réponse à l'administration du bolus, le fait de déterminer au moins l'une parmi une première valeur de paramètre de spectroscopie d'impédance électrochimique (EIS) ou une première valeur de conductivité en réponse à une exposition d'une électrode de travail (375) d'un capteur d'analyte à un analyte ; et
le fait de déterminer une présence d'un interférent en fonction d'au moins l'une parmi la première valeur de paramètre EIS ou la première valeur de conductivité.

10. Procédé mis en œuvre par processeur selon la revendication 9, comprenant en outre :
le fait d'ajuster une tension de polarisation de l'électrode de travail d'une première valeur de tension de polarisation à une seconde valeur de tension de polarisation en réponse à la présence de l'interférent, en particulier dans lequel la seconde valeur de tension de polarisation est inférieure à la première valeur de tension de polarisation.

11. Procédé mis en œuvre par processeur selon la revendication 9 ou 10, comprenant en outre :
le fait de déterminer au moins l'une parmi une seconde valeur de paramètre EIS ou une seconde valeur de conductivité en réponse à une exposition de l'électrode à l'analyte ; et
le fait de déterminer que l'interférent est inférieur à une quantité seuil en fonction d'au moins l'une parmi la seconde valeur de paramètre EIS ou la seconde valeur de conductivité,
comprenant en outre en particulier :
le fait d'ajuster la tension de polarisation de l'électrode de travail en réponse au fait que l'interférent est inférieur à la quantité seuil, en particulier dans lequel le fait d'ajuster la tension de polarisation de l'électrode de travail en réponse au fait que l'interférent est inférieur à la quantité seuil comporte la diminution progressive de la tension de polarisation.

12. Procédé mis en œuvre par processeur selon l'une des revendications 9 à 11, dans lequel la tension de polarisation est comprise dans une plage d'oxydation d'excipient d'insuline.

13. Procédé mis en œuvre par processeur selon l'une des revendications 9 à 12, dans lequel le fait de déterminer que la valeur d'analyte est supérieure au seuil est basé sur l'obtention d'une valeur de taille du bolus à partir de la pompe.

14. Support ou supports non transitoires lisibles par processeur stockant des instructions qui, lorsqu'elles sont exécutées par un ou plusieurs processeurs du capteur d'analyte (355) selon l'une des revendications 1 à 8, amènent la réalisation d'au moins :
l'obtention d'une indication provenant d'une pompe (530) selon laquelle un bolus est administré ;
en réponse à l'administration du bolus, le fait de déterminer au moins l'une parmi une première valeur de paramètre de spectroscopie d'impédance électrochimique (EIS) ou une première valeur de conductivité en réponse à une exposition d'une électrode de travail (375) d'un capteur d'analyte à un analyte ; et
le fait de déterminer une présence d'un interférent en fonction d'au moins l'une parmi la première valeur de paramètre EIS ou la première valeur de conductivité.
